# EUROPEAN PATENT SPECIFICATION

(11) **EP 2 050 761 B1**
(45) Date of publication and mention of the grant of the patent: **29.06.2011**
(21) Application number: 07118566.4
(22) Date of filing: 16.10.2007
(51) Int. Cl.: C07K 14/47, A61K 38/04, G01N 33/68

(54) **Galactosylated peptides, their preparation and use in autoimmune diseases diagnosis**
Galactosylierte Peptide, deren Zubereitung und Verwendung zur Diagnose von Autoimmunerkrankungen
Peptides galactosylés, leur préparation et utilisation pour le diagnostic de maladies auto-immunes

(43) Date of publication of application: 22.04.2009
(73) Proprietor: Toscana Biomarkers S.r.l., 53100 Siena (IT)
(72) Inventor: Alcaro, Maria Claudia, 53100 Siena (IT); Chelli, Mario, 50064 Incisa Val d'Arno (IT); Lolli, Francesco, 50127 Firenze (IT); Migliorini, Paola, 56122 Pisa (IT); Paolini, Ilaria, 53100 Siena (IT); Papini, Anna Maria, 50127 Firenze (IT); Rovero, Paolo, 50127 Firenze (IT)
(74) Representative: Gervasi, Gemma

(56) References cited:
- WO-A-03/000733
- A CAROTENUTO ET AL.: "Conformation-activity relationship of designed glycopeptides as synthetic probes for the detection of autoantibodies, biomarkers of multiple sclerosis" JOURNAL OF MEDICINAL CHEMISTRY., vol. 49, no. 17, 2006, pages 5072-5079, XP002475769 US AMERICAN CHEMICAL SOCIETY. WASHINGTON.
- F LOLLI ET AL.: "A N-glcosylated peptide detecting disease-specific autoantibodies, biomarkers of multiple sclerosis" PROCEEDINGS OF THE NATIONAL ACADEMY OF SCIENCES OF USA., vol. 102, no. 29, 19 July 2005 (2005-07-19), pages 10273-10278, XP002475770 US NATIONAL ACADEMY OF SCIENCE, WASHINGTON, DC.

### SEQUENCE LISTING - SHOWING AMENDMENTS

<110> TOSCANA BIOMARKERS S.r.l.
<120> Galactosylated peptides, their preparation and use in autoimmune diseases diagnosis
<130> 8492PTEP
<160> 163
<170> PatentIn version 3.3
<210> 1
   <211> 4
   <212> PRT
   <213> Artificial
<220>
   <223> Synthetic peptide
<220>
   <221> PEPTIDE
   <222> (1)..(4)
   <223> Formula (I): the residue in position 2 is an aminoacid carrying an aliphatic group on the side chain; the residue in position 3 may be: N, Q, Hyl, K, S, T; the residue in position 3 is galactosylated (Gal).
<400> 1
   Gly Xaa Xaa Gly
   1
<210> 20
   <211> 4
   <212> PRT
   <213> Artificial
<220>
   <223> Synthetic peptide
<220>
   <221> FPEPTIDE
   <222> (1)..(4)
   <223> Formula (II): the residue in position 1 may be: N, Q, Hyl, K, S, T. This residue is galactosylated (Gal); the residue in position 2 and 4 is an aminoacid other than P;
   the residue in position 3 may be S, T.
<400> 20
   Xaa Xaa Xaa Xaa
   1
<210> 33
   <211> 4
   <212> PRT
   <213> Artificial
<220>
   <223> Synthetic peptide
<220>
   <221> PEPTIDE
   <222> (1)..(4)
   <223> Formula (III): residue in position 1 is: D, N, R ,C, H, S, P, Y, G, L, V; residue in position 2 is: N, Q, Hyl, K, S, T and galactosylated (Gal); residue in position 3 is: D, N, S, T, G, V; residue in position 4 is: G, S, K, H, C, P, T.
<400> 33
   Xaa Xaa Xaa Xaa
   1
<210> 61
   <211> 4
   <212> PRT
   <213> Artificial
<220>
   <223> Synthetic peptide
<220>
   <221> PEPTIDE
   <222> (1)..(4)
   <223> Formula (IV): residue in position 1 may be: D, N, R, C, H, S, P, Y, G, L, V; residue in position 2 may be: S, P, K, D, H, N, G, E; residue in position 3 may be: N, Q, Hyl, K, S, T and galactosylated (Gal); residue in position 4 may be: G, S, K, H,
<400> 61
   Xaa Xaa Xaa Xaa
   1
<210> 122
   <211> 5
   <212> PRT
   <213> Artificial
<220>
   <223> Z2 chain
<220>
   <221> PEPTIDE
   <222> (1)..(5)
<400> 122
   Glu Pro Gly Ile Ala
   1               5
<210> 123
   <211> 5
   <212> PRT
   <213> Artificial
<220>
   <223> Z2 chain
<220>
   <221> MOD_RES
   <222> (1)..(5)
   <223> The residue in position 5 is 4Hyp
<220>
   <221> MOD_RES
   <222> (1)..(5)
   <223> The residue in position 5 is 4Hyp
<400> 123
   Thr Asp Gly Ile Xaa
   1               5
<210> 124
   <211> 5
   <212> PRT
   <213> Artificial
<220>
   <223> Z2 chain
<220>
   <221> PEPTIDE
   <222> (1)..(5)
<400> 124
   Thr Asp Gly Ile Pro
   1               5
<210> 125
   <211> 5
   <212> PRT
   <213> Artificial
<220>
   <223> Z2 chain
<220>
   <221> PEPTIDE
   <222> (1)..(5)
<400> 125
   Gln Asp Gly Leu Ala
   1 5
<210> 126
   <211> 5
   <212> PRT
   <213> Artificial
<220>
   <223> Z2 chain
<220>
   <221> PEPTIDE
   <222> (1)..(5)
<400> 126
   Pro Ser Gly Leu Ala
   1 5
<210> 127
   <211> 5
   <212> PRT
   <213> Artificial
<220>
   <223> Z2 chain
<220>
   <221> PEPTIDE
   <222> (1)..(5)
<400> 127
   Thr Pro Lys Val Gly
   1 5
<210> 128
   <211> 5
   <212> PRT
   <213> Artificial
<220>
   <223> Z2 chain
<220>
   <221> PEPTIDE
   <222> (1)..(5)
<400> 128
   Thr Pro Arg Ala Glu
   1 5
<210> 129
   <211> 5
   <212> PRT
   <213> Artificial
<220>
   <223> Z2 chain
<220>
   <221> PEPTIDE
   <222> (1)..(5)
<400> 129
   Thr Pro Arg Val Asp
   1 5
<210> 130
   <211> 5
   <212> PRT
   <213> Artificial
<220>
   <223> Z2 chain
<220>
   <221> PEPTIDE
   <222> (1)..(5)
<400> 130
   Thr Pro Lys Ala Glu
   1 5
<210> 131
   <211> 5
   <212> PRT
   <213> Artificial
<220>
   <223> Z2 chain
<220>
   <221> PEPTIDE
   <222> (1)..(5)
<400> 131
   Thr Glu Gly Ser Gln
   1 5
<210> 132
   <211> 5
   <212> PRT
   <213> Artificial
<220>
   <223> Z2 chain
<220>
   <221> PEPTIDE
   <222> (1)..(5)
<400> 132
   Arg Glu Glu Gln Tyr
   1 5
<210> 133
   <211> 5
   <212> PRT
   <213> Artificial
<220>
   <223> Z3 chain
<220>
   <221> PEPTIDE
   <222> (1)..(5)
<400> 133
   Glu Gln Gly Pro Ser
   1               5
<210> 134
   <211> 5
   <212> PRT
   <213> Artificial
<220>
   <223> Z3 chain
<220>
   <221> MOD_RES
   <222> (1)..(5)
   <223> the residue in position 4 is 4Hyp
<400> 134
   Glu Gln Gly Xaa Ser
   1               5
<210> 135
   <211> 5
   <212> PRT
   <213> Artificial
<220>
   <223> Z3 chain
<220>
   <221> PEPTIDE
   <222> (1)..(5)
<400> 135
   Ser Ala Gly Ala Pro
   1               5
<210> 136
   <211> 5
   <212> PRT
   <213> Artificial
<220>
   <223> Z3 chain
<220>
   <221> MOD_RES
   <222> (1)..(5)
   <223> the residue in position 5 is 4Hyp
<400> 136
   Ser Ala Gly Ala Xaa
   1 5
<210> 137
   <211> 5
   <212> PRT
   <213> Artificial
<220>
   <223> Z3 chain
<220>
   <221> MOD_RES
   <222> (1)..(5)
   <223> the residue in position 2 is 4Hyp
<400> 137
   Ala Xaa Gly Glu Arg
   1 5
<210> 138
   <211> 5
   <212> PRT
   <213> Artificial
<220>
   <223> Z3 chain
<220>
   <221> PEPTIDE
   <222> (1)..(5)
<400> 138
   Ala Pro Gly Glu Arg
   1 5
<210> 139
   <211> 5
   <212> PRT
   <213> Artificial
<220>
   <223> Z3 chain
<220>
   <221> PEPTIDE
   <222> (1)..(5)
<400> 139
   Ala Asn Gly Asp Pro
   1 5
<210> 140
   <211> 5
   <212> PRT
   <213> Artificial
<220>
   <223> Z3 chain
<220>
   <221> PEPTIDE
   <222> (1)..(5)
<400> 140
   Ser Ala Phe Ile Val
   1 5
<210> 141
   <211> 5
   <212> PRT
   <213> Artificial
<220>
   <223> Z3 chain
<220>
   <221> PEPTIDE
   <222> (1)..(5)
<400> 141
   Thr Ala Tyr Leu Val
   1 5
<210> 142
   <211> 5
   <212> PRT
   <213> Artificial
<220>
   <223> Z3 chain
<220>
   <221> PEPTIDE
   <222> (1)..(5)
<400> 142
   Arg Val Val Ser Val
   1 5
<210> 143
   <211> 5
   <212> PRT
   <213> Artificial
<220>
   <223> Z3 chain
<220>
   <221> PEPTIDE
   <222> (1)..(5)
<400> 143
   His Cys Lys Asn Ser
   1 5
<210> 144
   <211> 15
   <212> PRT
   <213> Artificial
<220>
   <223> Synthetic peptide
<220>
   <221> PEPTIDE
   <222> (1)..(15)
   <223> the residue in position 9 is galactosylated (Gal)
<400> 144
   Gly Glu Pro Gly Ile Ala Gly Phe Ser Gly Glu Gln Gly Pro Ser
   1 5 10 15
<210> 145
   <211> 15
   <212> PRT
   <213> Artificial
<220>
   <223> Synthetic peptide
<220>
   <221> PEPTIDE
   <222> (1)..(15)
   <223> the residue in position 9 is galactosylated (Gal)
<400> 145
   Gly Glu Pro Gly Ile Ala Gly Phe Asn Gly Glu Gln Gly Pro Ser
   1 5 10 15
<210> 146
   <211> 15
   <212> PRT
   <213> Artificial
<220>
   <223> Synthetic peptide
<220>
   <221> MOD_RES
   <222> (1)..(15)
   <223> the residue in position 9 is Hyl, this residue is galactosylated (Gal)
<400> 146
   Gly Glu Pro Gly Ile Ala Gly Phe Xaa Gly Glu Gln Gly Pro Ser
   1 5 10 15
<210> 147
   <211> 15
   <212> PRT
   <213> Artificial
<220>
   <223> Synthetic peptide
<220>
   <221> PEPTIDE
   <222> (1)..(15)
   <223> the residue in position 9 is galactosylated (Gal)
<400> 147
   Gly Thr Asp Gly Ile Pro Gly Leu Ser Gly Ser Ala Gly Ala Pro
   1               5                   10                  15
<210> 148
   <211> 15
   <212> PRT
   <213> Artificial
<220>
   <223> Synthetic peptide
<220>
   <221> PEPTIDE
   <222> (1)..(15)
   <223> the residue in position 9 is galactosylated (Gal)
<400> 148
   Gly Thr Asp Gly Ile Pro Gly Leu Asn Gly Ser Ala Gly Ala Pro
   1               5                   10                  15
<210> 149
   <211> 15
   <212> PRT
   <213> Artificial
<220>
   <223> Synthetic peptide
<220>
   <221> MOD_RES
   <222> (1)..(15)
   <223> the residue in position 9 is Hyl, this residue is galactosylated (Gal)
<400> 149
   Gly Thr Asp Gly Ile Pro Gly Leu Xaa Gly Ser Ala Gly Ala Pro
   1               5                   10                  15
<210> 150
   <211> 15
   <212> PRT
   <213> Artificial
<220>
   <223> Synthetic peptide
<220>
   <221> PEPTIDE
   <222> (1)..(15)
   <223> the residue in position 9 is Hyl, this residue is galactosylated (Gal)
<400> 150
   Gly Gln Asp Gly Leu Ala Gly Pro Ser Gly Ala Pro Gly Glu Arg
   1               5                   10                  15
<210> 151
   <211> 15
   <212> PRT
   <213> Artificial
<220>
   <223> Synthetic peptide
<220>
   <221> PEPTIDE
   <222> (1)..(15)
   <223> the residue in position 9 is galactosylated (Gal)
<400> 151
   Gly Gln Asp Gly Leu Ala Gly Pro Asn Gly Ala Pro Gly Glu Arg
   1               5                   10                  15
<210> 152
   <211> 15
   <212> PRT
   <213> Artificial
<220>
   <223> Synthetic peptide
<220>
   <221> MOD_RES
   <222> (1)..(15)
   <223> the residue in position 9 is Hyl, this residue is galactosylated (Gal)
<400> 152
   Gly Gln Asp Gly Leu Ala Gly Pro Xaa Gly Ala Pro Gly Glu Arg
   1               5                   10                  15
<210> 153
   <211> 15
   <212> PRT
   <213> Artificial
<220>
   <223> Synthetic peptide
<220>
   <221> PEPTIDE
   <222> (1)..(15)
   <223> the residue in position 9 is galactosylated (Gal)
<400> 153
   Gly Pro Ser Gly Leu Ala Gly Pro Ser Gly Ala Asn Gly Asp Pro
   1               5                   10                  15
<210> 154
   <211> 15
   <212> PRT
   <213> Artificial
<220>
   <223> Synthetic peptide
<220>
   <221> PEPTIDE
   <222> (1)..(15)
   <223> the residue in position 9 is galactosylated (Gal)
<400> 154
   Gly Pro Ser Gly Leu Ala Gly Pro Asn Gly Ala Asn Gly Asp Pro
   1               5                   10                  15
<210> 155
   <211> 15
   <212> PRT
   <213> Artificial
<220>
   <223> Synthetic peptide
<220>
   <221> MOD_RES
   <222> (1)..(15)
   <223> the residue in position 9 is Hyl, this residue is galactosylated (Gal)
<400> 155
   Gly Pro Ser Gly Leu Ala Gly Pro Xaa Gly Ala Asn Gly Asp Pro
   1               5                   10                  15
<210> 156
   <211> 16
   <212> PRT
   <213> Artificial
<220>
   <223> Synthetic peptide
<220>
   <221> PEPTIDE
   <222> (1)..(16)
   <223> the residue in position 7 is galactosylated (Gal)
<400> 156
   Pro Arg Glu Glu Gln Tyr Ser Ser Thr Tyr Arg Val Val Ser Val Leu
   1               5                   10                  15
<210> 157
   <211> 16
   <212> PRT
   <213> Artificial
<220>
   <223> Synthetic peptide
<220>
   <221> PEPTIDE
   <222> (1)..(16)
   <223> the residue in position 7 is galactosylated (Gal)
<400> 157
   Pro Arg Glu Glu Gln Tyr Asn Ser Thr Tyr Arg Val Val Ser Val Leu
   1 5 10 15
<210> 158
   <211> 16
   <212> PRT
   <213> Artificial
<220>
   <223> Synthetic peptide
<220>
   <221> PEPTIDE
   <222> (1)..(16)
   <223> the residue in position 7 is galactosylated (Gal)
<400> 158
   Ser Thr Glu Gly Ser Gln Ser Ile Thr Tyr His Cys Lys Asn Ser Ile
   1 5 10 15
<210> 159
   <211> 16
   <212> PRT
   <213> Artificial
<220>
   <223> Synthetic peptide
<220>
   <221> MOD_RES
   <222> (1)..(16)
   <223> the residue in position 7 is galactosylated (Gal)
<400> 159
   Ser Thr Glu Gly Ser Gln Asn Ile Thr Tyr His Cys Lys Asn Ser Ile
   1 5 10 15
<210> 160
   <211> 14
   <212> PRT
   <213> Artificial
<220>
   <223> Synthetic peptide
<220>
   <221> PEPTIDE
   <222> (1)..(14)
   <223> the residue in position 7 is galactosylated (Gal)
<400> 160
   Thr Pro Lys Ala Glu Arg Ser Ser Ala Phe Ile Val Pro Phe
   1 5 10
<210> 161
   <211> 14
   <212> PRT
   <213> Artificial
<220>
   <223> Synthetic peptide
<220>
   <221> PEPTIDE
   <222> (1)..(14)
   <223> the residue in position 7 is galactosylated (Gal)
<400> 161
   Thr Pro Lys Ala Glu Arg Asn Ser Ala Phe Ile Val Pro Phe
   1 5 10
<210> 162
   <211> 14
   <212> PRT
   <213> Artificial
<220>
   <223> Synthetic peptide
<220>
   <221> MOD_RES
   <222> (1)..(14)
   <223> the residue in position 7 is Hyl, this residue is galactosylated (Gal)
<400> 162
   Thr Pro Lys Ala Glu Arg Xaa Ser Ala Phe Ile Val Pro Phe
   1 5 10
<210> 163
   <211> 15
   <212> PRT
   <213> Artificial
<220>
   <223> Synthetic peptide
<220>
   <221> PEPTIDE
   <222> (1)..(15)
   <223> the residue in position 8 is galactosylated (Gal)
<400> 163
   Thr Pro Lys Ala Glu Arg Pro Asn Thr Ala Tyr Leu Val Pro Phe
   1 5 10 15

## Description

### Field of invention

The present invention refers to galactosylated peptides formed of 11-21 amino acids capable of identifying autoantibodies in autoimmune diseases such as Rheumatoid arthritis (RA), Juvenile RA, Systemic Lupus Erythematosus, Scleroderma, Sjogren's syndrome, and inflammatory bowel diseases, and therefore useful tools for diagnosis or therapeutic treatment of those pathologies, in particular RA.

### State of the art

RA, one of the most common and disabling autoimmune diseases, is characterized by inflammation of the synovial membrane of diarthrodial joints that can result in symmetrical joint inflammations, articular erosions and extra-articular complications.

Once that the acute synovite is caused from an exogenous agent, the autoimmune response is triggered against self antigens not more recognized as self and responsible of the chronicization of the disease.

Over the past 25 years, many autoantibody activities in RA have been described. In fact, abnormal antibodies in blood can be found in patients with RA.

RA and other rheumatic diseases are associated with a significant under expression of galactosyltransferase enzymes that results in a profound change in galactosylation of IgG. A number of studies clearly demonstrated that the occurrence of N-linked oligosaccharides lacking galactose is significantly higher in serum IgGs of patients with RA [A. Bond et al., Clin Exp Immunol, 1996, 105, 99]. These observations led to the hypothesis that this defect plays a crucial role in eliciting an autoimmune response in RA.

Rheumatoid factors (RFs) are antibodies directed to the Fc of IgG molecules, which can be found in 80% of patients. IgG RF has a self-binding capacity that can result in the formation of very large immune complexes, which are able to (further) activate the immune system. It is not clear whether RF is directly related to the pathogenesis of RA, although RF is found significantly more often in cases of aggressive joint inflammation. In those cases, RF titres are linearly related with the severity of inflammation. In fact, some studies showed that IgG molecules, containing less terminal galactose on their oligosaccharide moieties, are preferentially recognized by IgG and/or IgM RFs.

IgG has a conserved N-linked glycosylation site in the Fc region, with variable glycosylation in the Fab depending on the presence or absence of the glycosylation motif in the variable region and on its conformation. Investigation of serum IgG glycoforms has established significant differences in oligosaccharide structure between groups of patients with certain diseases compared with IgG from healthy controls.

The relationship between RF activity and glycosylation, however, is still a field of active investigation. Agalactosylated IgGs activate the complement pathway through binding to mannose binding lectin and are taken up by macrophages through the mannose receptor. Thus, it is likely that IgG-derived peptides associate with MHC class II molecules elicit an anti-IgG immune response, producing RFs. However, data so far available do not allow to precisely identify the glycosylated epitopes of IgG that are recognized by RF.

In 1964, a new RA-associated antibody termed antiperinuclear factor (APF) was described. This factor was the first of a new group of antibodies, which we currently know to be directed against citrullinated residues and known as anti-citrullinated protein/peptide antibodies (ACPA) [E.R. Vossenaar et al., Arthritis Res Ther 2004, 6, R142]. After the description of the APF, anti-keratin antibodies (AKAs, directed against epithelium of rat esophagus) were reported to be associated with RA. Moreover, it was shown that the epitopes recognised by both APFs and AKAs contain citrullinated residues, converted from arginine by the PAD enzyme. Since the epitope-specificity of ACPA was elucidated and, in order to optimize standardization of ACPA detection, more user-friendly tests were developed. Substrates for such tests include deiminated proteins (filaggrin or fibrinogen) and peptides [M. Sebbag et al., J Clin Invest 1995, 95, 2672; G. Serre. Joint Bone Spine 2001, 68, 103].

The most widely available synthetic peptide is known as CCP (cyclic citrullinated peptide). The first available CCP assay (anti-CCP1 [ER. Vossenaar et al., Clin Appl Immunol Rev 2004, 4, 239]) was further optimized by screening of dedicated peptide libraries (ImmunoscanRA Mark 2, manufactured by Euro-Diagnostica, Malmoe, Sweden; QuantaLite CCP2, manufactured by INOVA Diagnostics Inc., San Diego, CA).

Finally, another class of RA autoantibodies, directed against several types of collagens, has been investigated. Anticollagen antibodies are present in about 30% of RA patients. Although anti-type II collagen (anti-CII) autoantibodies can be found in serum of only a small proportion of RA patients, these antibodies and anti-CII-producing B cells are present in the joints of the majority of these patients. It is not clear whether the occurrence of anticollagen autoantibodies plays a role in the pathogenesis of RA or is merely a result of the disease, but it was recently demonstrated that the recognition of an immunodominant T cell epitope corresponding to the peptide CII(256-270), located within a polypeptide fragment of type II collagen which contains residues 256-270, plays a key role in triggering RA [J. Broddefalk et al., Org Chem 1999, 64, 8948; P. Kjelle'n et al., Eur. J. Immunol., 1998, 28, 755].

In particular, T cell receptors interact with galactosyl or glucosylgalactosyl molecules linked to Lys-264 and recent studies on synthetic glycosylated CII(256-270) peptides [J. Broddefalk et al., J. Am. Chem. Soc. 1998, 120, 7676] demonstrated the importance of this post-translational modification in T cell recognition.

In this scenario, the use of synthetic peptides chemically modified can be a useful technology for setting up diagnostic/prognostic tools based on recognition of antibodies and T cells, as disease biomarkers. This approach was successful applied in the field of Multiple Sclerosis (MS) by A.M. Papini et al. [PCT Application WO03/000733]. In fact, they showed that the presence of an aberrant N-glucosylation is possibly triggering an autoantibody response in MS and they detected for the first time autoantibodies in MS patients' sera by ELISA, using the synthetic peptide CSF114(Glc) characterized by a β-hairpin structure, optimally exposing on the tip of the β-turn the minimal epitope Asn(Glc) [F. Lolli et al., P.N.A.S. U.S.A. 2005, 102, 10273; F. Lolli et al., J. Neuroimmunology 2005, 167, 131; A. Carotenuto et al., J. Med. Chem. 2006, 49, 5072].

In a previous study [I. Paolini et al., Proceedings of the 29th European Peptide Symposium, online publication http://www.29eps.com/proceedings.asp, Paper no. 333] the possibility of identifying RA specific antibodies through glycosylated peptides has been proposed.

The interest in developing new glycosylated peptides capable of carrying out the function of identifying RA antibodies with greater efficiency and therefore useful both for the diagnosis and for the treatment of RA is evident.

In this context, the present invention refers to modified peptides as synthetic antigens for identifying RA specific autoantibodies, in order to develop versatile and simple diagnostic assays.

### Summary of the invention

It is therefore an object of the present invention to provide galactosylated peptides formed of 11-21 amino acids, comprising the following peptides sequences:

Z1-Z2-(G-X1-X(Gal)-G)-Z3 **(V)**

Z1-Z2-(X(Gal)-X2-S/T-X2)-Z3-Z4 **(VI)**

Z2-(X3-X(Gal)-X4-X5)-Z3-Z4-Z5 **(VII)**

Z2-(X3-X6-X(Gal)-X5)-Z3-Z4-Z5 **(VIII)**

Wherein
- in the sequence: - G-X1-X(Gal)-G (I) **SEQ ID N°:1**
   Gal= galactose
   X1 = amino acid carrying an aliphatic group on the side chain;
   X = N, Q, Hyl, K, S, T,
   wherein Hyl = 5-hydroxy-L-lysine;
- in the sequence: - X(Gal)-X2-S/T-X2 (II) **SEQ ID N°:20**
   X and Gal are as defined above;
   X2 = an amino acid other than P;
- in the sequence: - X3-X(Gal)-X4-X5 (III) **SEQ ID N°:33**
   X and Gal are as defined above;
   X3 = D, N, R, C, H, S, P, Y, G, L, V;
   X4 = D, N, S, T, G, V;
   X5 = G, S, K, H, C, P, T;
- in the sequence: - X3-X6-X(Gal)-X5 (IV) **SEQ ID N°:61**
   X, X3, X5 and Gal are as defined above;
   X6 = S, P, K, D, H, N, G, E.
   and wherein:
   Z1 is an amino acid selected from: G, P, S;
   Z2 is an amino acid sequence selected from:
   E P G I A **SEQ ID N°:122**
   T D G I O **SEQ ID N°:123**
   T D G I P **SEQ ID N°:124**
   Q D G L A **SEQ ID N°:125**
   P S G L A **SEQ ID N°:126**
   T P K V G **SEQ ID N°:127**
   T P R A E **SEQ ID N°:128**
   T P R V D **SEQ ID N°:129**
   T P K A E **SEQ ID N°:130**
   T E G S Q **SEQ ID N°:131**
   R E E Q Y; **SEQ ID N°:132**
   Z3 is an amino acid sequence selected from:
   E Q G P S **SEQ ID N°:133**
   EQGOS **SEQ ID N°:134**
   SAGAP **SEQ ID N°:135**
   SAGAO **SEQ ID N°:136**
   AOGER **SEQ ID N°:137**
   A P G E R **SEQ ID N°:138**
   A N G D P **SEQ ID N°:139**
   SAFIV **SEQ ID N°:140**
   TAYLV **SEQ ID N°:141**
   R V V S V **SEQ ID N°:142**
   H C K N S; **SEQ ID N°:143**
   Z4 is an amino acid selected from:
   P,L,I;
   Z5 is an amino acid selected from:
   Y, F, W.

### Detailed description of the invention

It has been surprisingly found, and is a subject of the present invention, that galactosylated peptides, formed of 11-21 amino acids as above defined, have a very efficient role in the recognition of autoantibodies typical of autoimmune diseases and are therefore useful tools for diagnosis or therapeutic treatment of those pathologies, in particular RA.

Peptide sequences, according to the present invention, were selected using protein alignment studies on collagen II (CII) and IgG primary structures taking into account the presence of N-glycosylation sites and the β-turn-forming tendencies of amino acids.

Especially preferred, according to the present invention, are the galactosylated peptides formed of 11-21 amino acids and characterized in order to contain at least an amino acid sequence selected from the following ones:

| | | |
|---|---|---|
| 1 | GEPGIAGFS(Gal)GEQGPS | SEQ ID N°:144 |
| 2 | GEPGIAGFN(Gal)GEQGPS | SEQ ID N°:145 |
| 3 | GEPGIAGFHyI(Gal)GEQGPS | SEQ ID N°:146 |
| 4 | GTDGIPGLS(Gal)GSAGAP | SEQ ID N°:147 |
| 5 | GTDGIPGLN(Gal)GSAGAP | SEQ ID N°:148 |
| 6 | GTDGIPGLHyI(Gal)GSAGAP | SEQ ID N°:149 |
| 7 | GQDGLAGPS(Gal)GAPGER | SEQ ID N°:150 |
| 8 | GQDGLAGPN(Gal)GAPGER | SEQ ID N°:151 |
| 9 | GQDGLAGPHyI(Gal)GAPGER | SEQ ID N°:152 |
| 10 | GPSGLAGPS(Gal)GANGDP | SEQ ID N°:153 |
| 11 | GPSGLAGPN(Gal)GANGDP | SEQ ID N°:154 |
| 12 | GPSGLAGPHyI(Gal)GANGDP | SEQ ID N°:155 |
| 13 | PREEQYS(Gal)STYRVVSVL | SEQ ID N°:156 |
| 14 | PREEQYN(Gal)STYRVVSVL | SEQ ID N°:157 |
| 15 | STEGSQS(Gal)ITYHCKNSI | SEQ ID N°:158 |
| 16 | STEGSQN(Gal)ITYHCKNSI | SEQ ID N°:159 |
| 17 | TPKAERS(Gal)SAFIVPF | SEQ ID N°:160 |
| 18 | TPKAERN(Gal)SAFIVPF | SEQ ID N°:161 |
| 19 | TPKAERHyI(Gal)SAFIVPF | SEQ ID N°:162 |
| 20 | TPKAERPN(Gal)TAYLVPF | SEQ ID N°:163 |

The peptides according to the present invention can be prepared according to the known methodologies for solution or solid-phase synthesis.

The solid-phase method, well known to the experts in the field, is especially useful. It consists of the covalent bond of the C-terminal residue to an appropriate solid support, such as polystyrene, polystyrene-polyoxyethylene or polyethylene glycol and polyacrylamide co-polymers. The successive amino acids are added sequentially, through acylation of the amino group of the residue bound to the resin. Upon completion of the synthesis, the crude peptide is obtained by treating the resin with an appropriate acid, for example trifluoroacetic acid, and separated by precipitation in ethyl ether and successive lyophilization. The peptide is finally purified by chromatographic techniques, such as preparative HPLC. It is also possible to maintain the synthetic peptide bound to the solid support, performing the selective deprotection of the side chains with an appropriate reagent.

An example describing the synthesis of galactosylated peptides according to the invention is reported here.

### Example 1. Peptide Synthesis

Peptides were synthetized on an automatic batch synthesizer, using a Wang resin preloaded with the C-terminal amino acid of the sequence, following the Fmoc/tBu solid-phase peptide synthesis strategy. The resin was swollen in DMF for 40 min. Fmoc deprotections were carried out in 30 min with 20% piperidine in DMF, and then washings with DMF were performed. Coupling reactions were performed by treating the resin for 45 min with a 0.5 M solution of the Fmoc-protected amino acids and HOBt in DMF (2.5 equiv), a 0.5 M solution of TBTU in DMF (2.5 equiv), and 4 M NMM in DMF (5 equiv). Coupling with Fmoc-Xaa[Gal(OPg)₄]-OH (Xaa = N, Hyl, S; Pg = Ac, Bz) was performed using HOBt, TBTU and NMM in DMF for 4 h.

Peptide cleavage from the resin and deprotection of the amino acid side chains were carried out in 3 h with TFA/thioanisole/EDT/phenol/H₂O (82.5:5:2.5:5:5) (vol:vol:vol:vol:vol). The resin was filtered off, and the solution was concentrated. The crude products were precipitated with cold Et₂O, centrifuged, and lyophilized. Deprotection of the sugar moiety was performed by adding 0.1 M MeONa to a solution of the crude material in dry MeOH to pH 12. After 2 h of stirring, the reaction was quenched with solid CO₂ to neutrality, the solvent was evaporated to dryness, and the residue was lyophilized.

The crude peptides were purified by HPLC and characterized by mass spectrometry.

Biological assays based on galactosylated peptides according to the invention, can be set up according to immunoenzymatic techniques, which consist on the immobilization on a solid surface of the peptide, used as antigenic molecule, by the adsorption or the covalent linkage of the peptide to the support.

### Adsorption-based techniques

Coating techniques based on the adsorption of the molecule proceed by the use of polystyrenic supports, generally used for immunoenzymatic assays, with a high, medium or low binding capacity for proteins and other molecules.

In the same way, peptides can be coated on appropriate supports, as chips, microspheres (gold, polystyrene, etc.), a wall of a reactor vessel or of a well of a microtitre plate, according to the standard techniques, to be used in diagnostics or therapeutics. These solid supports are generally functionalized with polymers, such as: silica gel, cellulose, polyacrylate, sepharose, and analogues, as well as the same resins normally used by the experts in the field for the preparation of synthetic peptides, such as polystyrene, polystyrene-polyoxyethylene or polyethylene glycol and polyacrylamide copolymers. Such polymers are derivatized with linkers or handles, which allow the formation of covalent bonds.

### Example 2. Adsorption-based ELISA

A peptide, prepared as described in example 1 and diluted in 0.05 M carbonate buffer (pH 9.6) was adsorbed onto 96-wells microtitre plates and left overnight at +4 °C. After plate washing (5 times with 0.05% v/v of Tween@20 in FBS), the wells were treated for 3 h at r.t. with FBS (FBS 10%, NaCl 9 g/L, Tween@20 0.05%). The patients' serum samples (diluted 1:100, 1:1000, 1:10000 in FBS buffer) were then loaded onto the plate overnight at +4 °C. After plate washing, an anti human-IgG/IgM antibody conjugated to the enzyme alkaline phosphatase was added and incubated for 1 h at r.t. After washings, the alkaline phosphate substrate p-nitrophenyl phosphate was added to the wells and the reaction was quenched after 30 min with 1 M NaOH. The absorbance was evaluated at a wavelength of 405 nm.

Serum samples of 52 patients suffering from RA, 12 patients suffering from multiple sclerosis, 8 healthy blood donors were tested by this method.

No antibody response was detected in sera of multiple sclerosis patients and healthy blood donors, while anti-galactosylated peptide antibodies were found in a 40% of RA patients sera.

### Example 3. Covalent ELISA

A peptide, prepared as described in example 1, was covalently linked to a 96-wells microtitre plate using N-hydroxysuccinimide (NHS), modified with a cationic or anionic group, and 1-ethyl-3-(3-dimethylaminopropyl)carbodiimide (EDC). Peptide coupling took place for 2 h at r.t. The plate was blocked for 1 h with 2% bovine serum albumin (BSA). The patients' serum samples were diluted 1:100, 1:1000, 1:10000 in FBS buffer (FBS 10%, NaCl 9g/L, Tween@20 0.05%) and were then loaded onto the plate for 3 h at r.t. After plate washing (3 times with 0.05% v/v of Tween@20 in FBS), an anti human-IgG/IgM antibody conjugated to the enzyme alkaline phosphatase was added and incubated for 1 h at r.t. After washings, the alkaline phosphate substrate p-nitrophenyl phosphate was added to the wells and the reaction was quenched after 30 min with 1 M NaOH. The absorbance was evaluated at a wavelength of 405 nm.

### Example 4. Conjugation of a peptide to resin

A peptide, prepared as described in example 1, was conjugated to sepharose resin preactivated with CNBr, according to the standard reaction protocols advised by the manufacturers in order to obtain a resin-peptide conjugate. The product thus obtained is useful as for example for the preparation of plates for the diagnosis or treatment of patients affected by RA.

The present invention refers also to a kit comprising the galactosylated peptides according to the invention and useful for diagnostic purposes.

According to a preferred embodiment of the invention, a kit as above mentioned comprises:
- a microplate;
- a galactosylated peptide according to the invention (lyophilized);
- a buffer solution for the adsorption or the covalent coupling of the peptide to the plate;
- FBS buffer (FBS 10%, NaCl 9 g/L, Tween@20 0.05%);
- concentrated wash solution;
- positive control serum;
- negative control serum;
- an antibody reacting with the antibody of RA [AP conjugated with anti-IgG/IgM];
- a substrate (p-nitrophenyl phosphate, disodium salt);
- a substrate buffer (1 M diethanolamine buffer, pH 9.8);
- a quenching solution (1 M sodium hydrate).

If preferred, the buffer solution for the adsorption of the peptide to the plate and the galactosylated peptide according to the invention can be incorporated directly in the microplate.

### Diagnostic/prognostic use

For diagnostic/prognostic use the galactosylated peptides according to the invention were diluted and adsorbed or covalently linked onto polymeric surfaces. Patients' serum or plasma was then added in a series of different concentration (dilution series). The autoantibody specific for our products binds to the peptide adsorbed or linked onto the polymer. The autoantibody molecules bound to the galactopeptides are then evidenced through the binding of appropriate secondary antibodies, which recognize the immunoglobulin constant fragment. These secondary antibodies, conjugated with appropriate enzymes, can be visualized through a colorimetric reaction: the absorbance developed is proportional to the amount of specifically bound autoantibody. Quantitatively, the result is expressed as the antibody titre, defined as the reciprocal of the dilution factor in which no further reaction is observed.

### Therapeutic use

The peptides according to the invention, in free form or bound to appropriate resins, can be used for the treatment of patients affected by RA as, thanks to their high specificity of antibody recognition, they can be used to selective antibody removal and also immunomodulation of the disease.

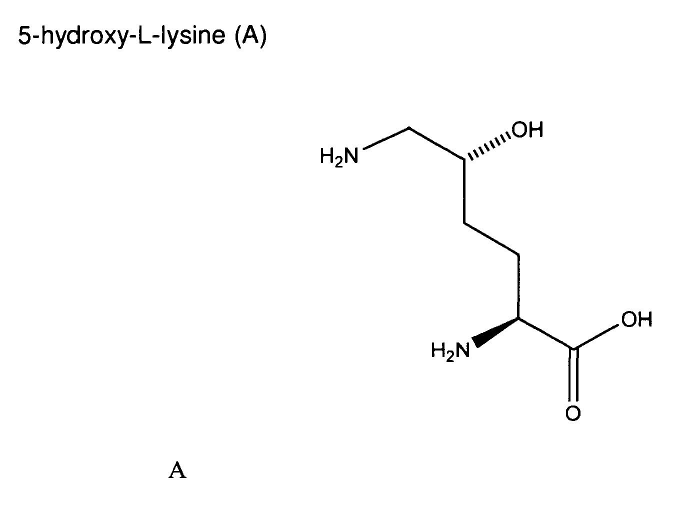

## Claims

1. Galactosylated peptides consisting of 11-21 amino acids comprising the following peptides sequences:
Z1-Z2-(G-X1-X(Gal)-G)-Z3 **(V)**
Z1-Z2-(X(Gal)-X2-S/T-X2)-Z3-Z4 **(VI)**
Z2-(X3-X(Gal)-X4-X5)-Z3-Z4-Z5 **(VII)**
Z2-(X3-X6-X(Gal)-X5)-Z3-Z4-Z5 **(VIII)**
Wherein
- in the sequence: - G-X1-X(Gal)-G (I) **SEQ ID N°:1**
Gal= galactose
X1 = amino acid carrying an aliphatic group on the side chain;
X = N, Q, Hyl, K, S, T,
wherein Hyl = 5-hydroxy-L-lysine;
- in the sequence: - X(Gal)-X2-S/T-X2 (II) **SEQ ID N°:20**
X and Gal are as defined above;
X2 = an amino acid other than P;
- in the sequence: - X3-X(Gal)-X4-X5 (III) **SEQ ID N°:33**
X and Gal are as defined above;
X3 = D, N, R, C, H, S, P, Y, G, L, V;
X4 = D, N, S, T, G, V;
X5 = G, S, K, H, C, P, T;
- in the sequence: - X3-X6-X(Gal)-X5 (IV) **SEQ ID N°:61**
X, X3, X5 and Gal are as defined above;
X6 = S, P, K, D, H, N, G, E.
and wherein:
Z1 is an amino acid selected from: G, P, S;
Z2 is an amino acid sequence selected from:
E P G I A **SEQ ID N°:122**
T D G I O **SEQ ID N°:123**
T D G I P **SEQ ID N°:124**
Q D G LA **SEQ ID N°:125**
P S G L A **SEQ ID N°:126**
T P K V G **SEQ ID N°:127**
T P R A E **SEQ ID N°:128**
T P R V D **SEQ ID N°:129**
T P K A E **SEQ ID N°:130**
T E G S Q **SEQ ID N°:131**
REEQY; **SEQ ID N°:132**
Z3 is an amino acid sequence selected from:
E Q G P S **SEQ ID N°:133**
EQGOS **SEQ ID N°:134**
SAGAP **SEQ ID N°:135**
SAGAO **SEQ ID N°:136**
AOGER **SEQ ID N°:137**
A P G E R **SEQ ID N°:138**
A N G D P **SEQ ID N°:139**
SAFIV **SEQ ID N°:140**
TAYLV **SEQ ID N°:141**
RVVSV **SEQ ID N°:142**
H C K N S; **SEQ ID N°:143**
Z4 is an amino acid selected from:
P,L,I;
Z5 is an amino acid selected from:
Y, F, W.

2. Galactosylated peptides according to Claims 1 chosen in the group consisting of:
GEPGIAGFS(Gal)GEQGPS **SEQ ID N°:144**
GEPGIAGFN(Gal)GEQGPS **SEQ ID N°:145**
GEPGIAGFHyI(Gal)GEQGPS **SEQ ID N°:146**
GTDGIPGLS(Gal)GSAGAP **SEQ ID N°:147**
GTDGIPGLN(Gal)GSAGAP **SEQ ID N°:148**
GTDGIPGLHyI(Gal)GSAGAP **SEQ ID N°:149**
GQDGLAGPS(Gal)GAPGER **SEQ ID N°:150**
GQDGLAGPN(Gal)GAPGER **SEQ ID N°:151**
GQDGLAGPHyI(Gal)GAPGER **SEQ ID N°:152**
GPSGLAGPS(Gal)GANGDP **SEQ ID N°:153**
GPSGLAGPN(Gal)GANGDP **SEQ ID N°:154**
GPSGLAGPHyI(Gal)GANGDP **SEQ ID N°:155**
PREEQYS(Gal)STYRVVSVL **SEQ ID N°:156**
PREEQYN(Gal)STYRVVSVL **SEQ ID N°:157**
STEGSQS(Gal)ITYHCKNSI **SEQ ID N°:158**
STEGSQN(Gal)ITYHCKNSI **SEQ ID N°:159**
TPKAERS(Gal)SAFIVPF **SEQ ID N°:160**
TPKAERN(Gal)SAFIVPF **SEQ ID N°:161**
TPKAERHyI(Gal)SAFIVPF **SEQ ID N°:162**
TPKAERPN(Gal)TAYLVPF **SEQ ID N°:163**

3. Process for the preparation of the galactosylated peptides according to Claim 1 according to the methodologies for solution synthesis.

4. Process for the preparation of the galactosylated peptides according to the methodologies of solid-phase synthesis.

5. Conjugates comprising a solid support and a galactosylated peptide according to claims 1 - 2.

6. Conjugates according to Claim 5 wherein said solid support is chosen in the group consisting of: polystyrene, polystyrene-polyoxyethylene or polyethylene glycol and polyacrylamide co-polymers.

7. Use of the galactosylated peptides according to Claim 1 - 6 as in vitro diagnostic for identifying autoantibodies in autoimmune diseases wherein said autoimmune disease is Rheumatoid Arthritis (RA).

8. Use of the galactosylated antibodies according to Claim 1 - 6 for the preparation of pharmaceutical composition for the treatment of autoimmune diseases diseases wherein said autoimmune disease is Rheumatoid Arthritis (RA).

## Patentansprüche

1. Galaktosylierte Peptide, bestehend aus 11-21 Aminosäuren, umfassend die folgenden Peptidsequenzen:
Z1-Z2-(G-X1-X(Gal)-G)-Z3 **(V)**
Z1-Z2-(X(Gal)-X2-S/T-X2)-Z3-Z4 **(VI)**
Z2-(X3-X(Gal)-X4-X5)-Z3-Z4-Z5 **(VII)**
Z2-(X3-X6-X(Gal)-X5)-Z3-Z4-Z5 **(VIII)**
wobei
- in der Sequenz: -G-X1-X(Gal)-G (I) **SEQ ID NO:1**
Gal = Galaktose
X1 = Aminosäure mit einer aliphatischen Gruppe an der Seitenkette
X = N, Q, Hyl, K, S, T
wobei Hyl = 5-Hydroxy-L-Lysin
- in der Sequenz: -X(Gal)-X2-S/T-X2 (II) **SEQ. ID NO.:20**
X und Gal, wie oben definiert sind:
X2 = eine andere Aminosäure als P ist;
- in der Sequenz: -X3-X(Gal)-X4-X5 (III) **SEQ ID NO.:33**
X und Gal wie oben definiert sind;
X3 = D, N, R, C, H, S, P, Y, G, L, V;
X4 = D, N, S, T, G, V
X5 = G, S, K, H, C, P, T;
- in der Sequenz: -X3-X6-X(Gal)-X5 (IV) **SEQ ID No.61** X, X3, X5 und Gal wie oben definiert sind;
X6 = S, P, K, D, H, N, G, E ist
und wobei:
Z1 eine Aminosäure ist, ausgewählt aus: G, P, S;
Z2 eine Aminosäuresequenz ist, ausgewählt aus:
EPGIA **SEQ ID No.122**
TDGIO **SEQ ID No 123**
TDGIP **SEQ ID No.124**
QDGLA **SEQ ID No.125**
P S G L A **SEQ ID No.126**
TPKVG **SEQ ID No.127**
TPRAE **SEQ ID No.128**
T P R V D **SEQ ID No.129**
TPKAE **SEQ ID No.130**
TEGSQ **SEQ ID No.131**
REEQY **SEQ ID No.132**
Z3 eine Aminosäuresequenz ist, ausgewählt aus:
EQGPS **SEQ ID No.133**
EQGOS **SEQ ID No.134**
SAGAP **SEQ ID No.135**
SAGAO **SEQ ID No.136**
AOGER **SEQ ID No.137**
APGER **SEQ ID No.138**
ANGDP **SEQ ID No.139**
S A F I V **SEQ ID No.140**
T A Y L V **SEQ ID No.141**
R V V S V **SEQ ID No.142**
HCKNS **SEQ ID No.143**
Z4 eine Aminosäure ist, ausgewählt aus:
P, L, I;
Z5 eine Aminosäure ist, ausgewählt aus:
Y, F, W;

2. Galaktosylierte Peptide nach Anspruch 1, ausgewählt aus der Gruppe, bestehend aus:
GEPGIAGFS(Gal)GEQGPS **SEQ ID No.144**
GEPGIAGFN(Gal)GEQGPS **SEQ ID No.145**
GEPGIAGFHyl(Gal)GEQGPS **SEQ ID No.146**
GTDGIPGLS(Gal)GSAGAP **SEQ ID No.147**
GTDGIPGLN(Gal)GSAGAP **SEQ ID No.148**
GTDGIPGLHyl(Gal)GSAGAP **SEQ ID No.149**
GQDGLAGPS(Gal)GAPGER **SEQ ID No.150**
GQDGLAGPN(Gal)GAPGER **SEQ ID No.151**
GQDGLAGPHyl(Gal)GAPGER **SEQ ID No.152**
GPSGLAGPS(Gal)GANGDP **SEQ ID No.153**
GPSGLAGPN(Gal)GANGDP **SEQ ID No.154**
GPSGLAGPHyl(Gal)GANGDP **SEQ ID No.155**
PREEQYS(Gal)STYRVVSVL **SEQ ID No.156**
PREEQYN(Gal)STYRVVSVL **SEQ ID No.157**
STEGSQS(Gal)ITYHCKNSI **SEQ ID No.158**
STEGSQN(Gal)ITYHCKNSI **SEQ ID No.159**
TPKAERS(Gal)SAFIVPF **SEQ ID No.160**
TPKAERN(Gal)SAFIVPF **SEQ ID No.161**
TPKAERHyI(Gal)SAFIVPF **SEQ ID No.162**
TPKAERPN(Gal)TAYLVPF **SEQ ID No.163**

3. Verfahren zur Herstellung der galaktosylierten Peptide nach Anspruch 1 gemäß den Verfahren für die Lösungssynthese.

4. Verfahren zur Herstellung der galaktosylierten Peptide gemäß den Verfahren der Festphasensynthese.

5. Konjugate umfassend einen festen Träger und ein galaktosyliertes Peptid nach Ansprüchen 1-2.

6. Konjugate nach Anspruch 5, wobei genannter fester Träger aus der Gruppe ausgewählt ist, bestehend aus: Polystyrol, Polystyrol-polyoxyethylen oder Polyethylenglycol und Polyacrylamid-Co-Polymeren.

7. Verwenden der galaktosylierten Peptide nach Anspruch 1-6 zur In-Vitro-Diagnostik für die Indentifizierung von Autoantikörpern bei Automimmunkrankheiten, wobei die genannte Autoimmunkrankheit rheumatoide Arthritis ist (RA).

8. Verwenden der galaktosylierten Antikörper nach Ansprüchen 1-6 zur Herstellung einer pharmazeutischen Zusammensetzung für die Behandlung von Autoimmunerkrankungen, wobei die genannte Autoimmunkrankheit rheumatoide Arthritis (RA) ist.

## Revendications

1. Peptides galactosylés constitués de 11 à 21 acides aminés comprenant les séquences peptidiques suivantes :
Z1-Z2-(G-X1-X(Gal)-G)-Z3 (V)
Z1-Z2-(X(Gal)-X2-S/T-X2)-Z3-Z4 (VI)
Z2-(Z3-X(Gal)-X4-X5)-Z3-Z4-Z5 (VII)
Z2-(X3-X6-X(Gal)-X5)-Z3-Z4-Z5 (VIII)
dans lesquelles
dans la séquence : -G-X1-X(Gal)-G (I) SEQ ID NO : 1
Gal = galactose
X1 = acide aminé portant un groupe aliphatique sur la chaîne latérale ;
X = N, Q, Hyl, K, S, T,
où Hyl = 5-hydroxy-L-lysine ;
dans la séquence : -X(Gal)-X2-S/T-X2 (II) SEQ ID NO : 20
X et Gal sont tels que définis ci-dessus ;
X2 = un acide aminé différent de P ;
dans la séquence : -X3-X(Gal)-X4-X5 (III) SEQ ID NO : 33
X et Gal sont tels que définis ci-dessus ;
X3 = D, N, R, C, H, S, P, Y, G, L, V ;
X4 = D, N, S, T, G, V ;
X5 = G, S, K, H, C, P, T ;
dans la séquence : -X3-X6-X(Gal)-X5 (IV) SEQ ID NO : 61
X, X3, X5 et Gal sont tels que définis ci-dessus ;
X6 = S, P, K, D, H, N, G, E,
et dans lesquelles :
Z1 représente un acide aminé choisi parmi : G, P, S ;
Z2 représente une séquence d'acides aminés choisie parmi :
E P G I A SEQ ID NO : 122
T D G I O SEQ ID NO : 123
T D G I P SEQ ID NO : 124
Q D G L A SEQ ID NO : 125
P S G L A SEQ ID NO : 126
T P K V G SEQ ID NO : 127
T P R A E SEQ ID NO : 128
T P R V D SEQ ID NO : 129
T P K A E SEQ ID NO : 130
T E G S Q SEQ ID NO : 131
R E E Q Y SEQ ID NO : 132
Z3 représente une séquence d'acides aminés choisie parmi :
E Q G P S SEQ ID NO : 133
E Q G O S SEQ ID NO : 134
S A G A P SEQ ID NO : 135
S A G A O SEQ ID NO : 136
A O G E R SEQ ID NO : 137
A P G E R SEQ ID NO : 138
A N G D P SEQ ID NO : 139
S A F I V SEQ ID NO : 140
T A Y L V SEQ ID NO : 141
R V V S V SEQ ID NO : 142
H C K N S SEQ ID NO : 143
Z4 représente un acide aminé choisi parmi :
P, L, I ;
Z5 représente un acide aminé choisi parmi ;
Y, F, W.

2. Peptides galactosylés selon la revendication 1, choisis dans le groupe comprenant :
G E P G I A G F S (Gal) G E Q G P S SEQ ID NO : 144
G E P G I A G F N (Gal) G E Q G P S SEQ ID NO : 145
G E P G I A G F Hyl(Gal) G E Q G P S SEQ ID NO : 146
G T D G I P G L S(Gal) G S A G A P SEQ ID NO : 147
G T D G I P G L N(Gal) G S A G A P SEQ ID NO : 148
G T D G I P G L Hyl(Gal) G S A G A P SEQ ID NO : 149
G Q D G L A G P S (Gal) G A P G E R SEQ ID NO : 150
G Q D G L A G P N (Gal) G A P G E R SEQ ID NO : 151
G Q D G L A G P Hyl(Gal) G A P G E R SEQ ID NO : 152
G P S G L A G P S(Gal) G A N G D P SEQ ID NO : 153
G P S G L A G P N(Gal) G A N G D P SEQ ID NO : 154
G P S G L A G P Hyl(Gal) G A N G D P SEQ ID NO : 155
P R E E Q Y S(Gal) S T Y R V V S V L SEQ ID NO : 156
P R E E Q Y N(Gal) S T Y R V V S V L SEQ ID NO : 157
S T E G S Q S(Gal) I T Y H C K N S I SEQ ID NO : 158
S T E G S Q N(Gal) I T Y H C K N S I SEQ ID NO : 159
T P K A E R S(Gal) S A F I V P F SEQ ID NO : 160
T P K A E R N(Gal) S A F I V P F SEQ ID NO : 161
T P K A E R Hyl(Gal) S A F I V P F SEQ ID NO : 162
T P K A E R P N(Gal) T A Y L V P F SEQ ID NO : 163

3. Procédé de préparation des peptides galactosylés selon la revendication 1, selon les méthodologies pour la synthèse en solution.

4. Procédé de préparation des peptides galactosylés, selon les méthodologies de la synthèse sur phase solide.

5. Conjugués comprenant un support solide et un peptide galactosylé selon les revendications 1 ou 2.

6. Conjugués selon la revendication 5, où ledit support solide est choisi dans le groupe comprenant : le polystyrène, le polystyrène-polyoxyéthylène, ou des copolymères de polyéthylène glycol et de polyacrylamide.

7. Utilisation des peptides galactosylés selon les revendications 1 à 6, en tant que diagnostic *in vitro* pour identifier des auto-anticorps dans des maladies auto-immunes où ladite maladie auto-immune est la polyarthrite rhumatoïde (PR).

8. Utilisation des anticorps galactosylés selon les revendications 1 à 6 pour la préparation d'une composition pharmaceutique destinée au traitement de maladies auto-immunes où ladite maladie auto-immune est la polyarthrite rhumatoïde (PR).
